# EUROPEAN PATENT APPLICATION

(11) **EP 4 778 623 A1**
(43) Date of publication of application: **22.07.2026**
(21) Application number: 24864600.2
(22) Date of filing: 10.09.2024
(51) Int. Cl.: B01J 19/00

(54) **FULLY AUTOMATIC OLIGONUCLEOTIDE SYNTHESIS DEVICE AND METHOD**

(30) Priority: 12.09.2023 CN 202311172147
(71) Applicant: WUXI APPTEC (TIANJIN) CO., LTD., Tianjin 300457 (CN); Changzhou Syntheall Pharmaceuticals Co., Ltd., Changzhou, Jiangsu 213001 (CN)
(72) Inventor: LI, Zhemeng, Tianjin 300457 (CN); WEI, Zhanlei, Tianjin 300457 (CN); YANG, Yun, Tianjin 300457 (CN)
(74) Representative: Cabinet Becker et Associés
(86) International application number: PCT/CN2024/117938
(87) International publication number: WO 2025/055881

(57) **Abstract**

A fully automatic oligonucleotide synthesis device, comprising: a pipeline liquid feeding system, a reaction system and a control system, wherein the pipeline liquid feeding system comprises a pumping module, a moving module, and a liquid injection module, the liquid injection module being mounted on the moving module, and the moving module being configured to enable the liquid injection module to move three-dimensionally to inject liquid into the reaction system; and the control system is in communication connection with the pipeline liquid feeding system and the reaction system and is used for controlling the liquid feeding and reaction. The device preferably further comprises a non-pipeline liquid feeding system, wherein the non-pipeline liquid feeding system comprises a pipetting apparatus, and the pipetting apparatus is preferably mounted on the moving module and is used for injecting the liquid into the reaction system. In addition, further provided is a method for synthesizing oligonucleotides by using the fully automatic oligonucleotide synthesis device. The fully automatic oligonucleotide synthesis device has a high degree of automation and is suitable for oligonucleotide synthesis of different scales, thereby achieving high synthesis quality and high efficiency.

## Description

### Technical Field

The present application belongs to the field of gene synthesis, specifically relating to fully automatic oligonucleotide synthesis devices and methods, especially fully automated synthesis devices and methods for phosphorodiamidate morpholino oligomers (PMOs).

### Background Art

PMO has a high affinity for DNA and RNA, is resistant to various nucleases, is stable in vivo, and has low toxicity, making it a highly anticipated compound as an antisense oligonucleotide.

PMO synthesis is mainly carried out artificially, and there is currently no mature PMO synthesizer on the market. Chengxi Li et al. (Fully automated fast-flow synthesis of antisense phosphorodiamidate morpholino oligomers, Nature Communications, volume 12, Article number: 4396 (2021)) discloses a rapid PMO synthesis reactor that can complete the synthesis of a single sequence in 3.5 h, but it features a small synthesis scale (<1µmol) and is incapable of parallel batch synthesis. Chinese patent CN 217829968U discloses a laboratory oligonucleotide synthesizer, Chinese patent application CN 114984896A discloses an oligonucleotide synthesis device, and Chinese patent application CN 114985023A discloses a microarray chip and an oligonucleotide synthesis device including the microarray chip. However, they have small synthesis scale, poor automation, and cannot achieve full automation of the production process.

The synthesis devices currently used for PMO synthesis suffer from the following drawbacks: lack of full-process automation in production, where human-induced variables introduce potential risks of production failure; inconsistent synthesis quality, poor reproducibility, and high monomer consumption, which preclude large-scale parallel synthesis; absence of an inert gas protection mode and an efficient exhaust system as well as poor airtightness, leading to easy solvent volatilization and strong odors in the production environment; exposed product handling operations, which not only cause intense solvent odors but also raise risks of product loss due to spillage and foreign matter contamination.

Therefore, there is a need in the art for improved fully automatic oligonucleotide synthesis devices and methods.

### Summary of the Invention

An objective of the present disclosure is to provide a novel automatic oligonucleotide synthesis device to solve the above-mentioned problems, which can achieve high-throughput, large-scale rapid synthesis of PMOs.

In an aspect, the present disclosure provides a fully automatic oligonucleotide synthesis device, and the device comprises: a pipeline liquid feeding system, a reaction system and a control system, wherein the pipeline liquid feeding system comprises a pumping module, a moving module, and a liquid injection module, the liquid injection module being mounted on the moving module, and the moving module being configured to enable the liquid injection module to move three-dimensionally to inject liquid into the reaction system; and the control system is in communication connection with the pipeline liquid feeding system and the reaction system and is used for controlling the liquid feeding and reaction.

In some embodiments, the device further comprises a non-pipeline liquid feeding system, the non-pipeline liquid feeding system comprises a pipetting apparatus, and the pipetting apparatus is preferably mounted on the moving module and is used for injecting the liquid into the reaction system.

In some embodiments, the reaction system comprises at least one reaction module, preferably at least two reaction modules, such as 3, 4, 5 or 6 reaction modules; each reaction module comprises at least one reactor, preferably at least two reactors, such as 3, 4, 5 or 6 reactors.

In some embodiments, each reactor is housed in an adapter sleeve.

In some embodiments, the reaction module comprises a shaking mechanism, the shaking mechanism preferably comprises a motion guide rail, more preferably two sets of motion guide rails configured to shake the reactor.

In some embodiments, the device comprises one or more liquid injection modules, each liquid injection module comprises a liquid injection port for injecting liquid into the reactor, preferably the number of liquid injection ports of each liquid injection module is equal to the number of reactors of each reaction module.

In some embodiments, the moving module comprises a first guide rail, a second guide rail and a third guide rail that are perpendicular to each other, the second guide rail is configured to move horizontally along the first guide rail; the third guide rail is mounted on the second guide rail and is configured to move horizontally along the second guide rail.

In some embodiments, the liquid injection module is mounted on the third guide rail and is configured to move vertically up and down along the third guide rail.

In some embodiments, the pumping module comprises an electric injector and a solenoid valve, wherein the electric injector and the solenoid valve are connected via a pipeline, the solenoid valve comprises an input port and an output port, the input port is connected to a reagent reservoir via a pipeline, and the output port is connected to the liquid injection module via a pipeline.

In some embodiments, the device comprises a plurality of reagent reservoirs, wherein each of at least some of the reagent reservoirs is provided with one or more reagent outlets, and each reagent outlet is connected to the input port of a solenoid valve via a pipeline.

In some embodiments, at least one reagent reservoir comprises only one reagent outlet, the reagent outlet is connected to the input port of a solenoid valve, there is a flow divider between the output port of the solenoid valve and the liquid injection module, the flow divider comprises a flow divider inlet and a plurality of flow divider outlets, the flow divider inlet is connected to the output port of the solenoid valve via a pipeline, and each flow divider outlet is connected to a liquid injection port via a pipeline.

In some embodiments, at least one of the plurality of reagent reservoirs is configured to allow a pipetting apparatus to transfer reagents.

In another aspect, the present disclosure provides a method for synthesizing oligonucleotides by using the fully automatic oligonucleotide synthesis device, comprising
a. washing a reactor;
b. adding a deprotecting agent to the reactor to carry out a deprotection reaction;
c. washing the reactor;
d. adding a neutralizing agent to the reactor to carry out a neutralization reaction;
e. washing the reactor;
f. adding a coupling unit to the reactor;
g. carrying out a coupling reaction,
wherein under the control of a control system, steps a to h are executed cyclically until the desired oligonucleotide is obtained, the method comprising, under the control of the control system, moving a liquid injection module using a moving module to inject the reagent into the reactor.

In some embodiments, the method for synthesizing oligonucleotides further comprises, under the control of the control system, using the pipetting apparatus to transfer and inject the reagent into the reactor, wherein the liquid injection module and the pipetting apparatus are used for injecting different reagents.

In some embodiments, the fully automatic oligonucleotide synthesis device comprises a plurality of reaction modules, after step f is completed in one reaction module and during the execution of step g, steps a to f are performed on the next reaction module until steps a to f are completed in all the required number of reaction modules.

In some embodiments, one or more of the processes of washing, deprotection reaction, neutralization reaction, and coupling reaction, preferably all of the processes, involve one or more of the following operations under the control of the control system:
shaking the reactor;
heating the reactor to a predetermined temperature and maintaining it at that predetermined temperature;
blowing nitrogen into the reactor for isolation from air.

In some embodiments, the oligonucleotide is a phosphorodiamidate morpholino oligomer.

In some embodiments, in step a, the reactor is washed with dichloromethane (DCM) and 30% trifluoroethanol (TFE)/dichloromethane (DCM) in sequence, preferably using the liquid injection module for addition, and each washing is preferably carried out for 30 s to 5 min, for example, 1 min.

In some embodiments, in step b, the deprotecting agent is a solution of trifluoroacetic acid (TFA) and 4-cyanopyridine in trifluoroethanol (TFE)/dichloromethane, preferably using the liquid injection module for addition, and the reaction is preferably carried out for 10 to 20 min, for example, 15 min.

In some embodiments, in step c, the reactor is washed with dichloromethane, preferably using the liquid injection module for addition, and the washing is preferably carried out for 30 s to 5 min, for example, 1 min.

In some embodiments, in step d, the neutralizing agent is a mixed solution of N,N-diisopropylethylamine (DIEA), isopropanol and dichloromethane, preferably using the liquid injection module for addition, and the reaction is preferably carried out for 30 s to 5 min, for example, 1 min.

In some embodiments, in step e, the reactor is washed with dichloromethane and 1,3-dimethyl-2-imidazolidinone (DMI) in sequence, preferably using the liquid injection module for addition, and each washing is preferably carried out for 30 s to 5 min, for example, 1 min.

In some embodiments, in step f, a 10% by volume solution of N-ethylmorpholine in 1,3-dimethyl-2-imidazolidinone (DMI) and a 0.1M solution of a phosphorodiamidate morpholino oligomer monomer in 1,3-dimethyl-2-imidazolidinone (DMI) are added to the reactor, preferably using the pipetting apparatus for addition.

In some embodiments, in step g, the reaction lasts for 2 to 4 h, for example, 3 h.

### Brief Description of the Drawings

To more clearly illustrate the technical solution of the present application, the accompanying drawings involved in some embodiments of the present application will be briefly described below. It is self-evident that the embodiments illustrated in the accompanying drawings below are merely exemplary; for a person of ordinary skill in the art, other drawings may be derived from the said drawings without exerting any creative effort.
Fig. 1 is a partial schematic diagram of a fully automatic oligonucleotide synthesis device according to some embodiments of the present disclosure.
Fig. 2A is a schematic diagram of a pumping module according to some embodiments of the present disclosure.
Fig. 2B is a partial schematic diagram of a pumping module according to some embodiments of the present disclosure.
Fig. 3A is a schematic diagram of a moving module according to some embodiments of the present disclosure.
Fig. 3B is a schematic diagram of a pipette according to some embodiments of the present disclosure.
Fig. 4A is a schematic diagram of a reaction module according to some embodiments of the present disclosure.
Fig. 4B is an exploded schematic diagram of a reaction tube, a reaction tube adapter sleeve and a reaction tube seat according to some embodiments of the present disclosure.
Fig. 4C is a schematic diagram of an assembly formed by assembling the reaction tube, reaction tube adapter sleeve and reaction tube seat shown in Fig. 4B.
Fig. 4D is a perspective view of the assembly shown in Fig 4C.
Fig. 5A is a schematic diagram showing the distribution of liquid injection ports according to some embodiments of the present disclosure.
Fig. 5B is a schematic diagram of a liquid injection module according to some embodiments of the present disclosure.
Fig. 6 shows the test results of the phosphorodiamidate morpholino oligomers prepared in the examples of the present disclosure.

List of Reference Numerals:
1-1: Pumping module; 1-2: Reaction module; 1-3: Liquid injection module; 1-4: Moving module; 1-5: Pipetting apparatus/pipette; 2-1: Solenoid valve; 2-2: Electric injector; 2-3: Lead screw; 2-4: Coupling; 2-5: Pumping motor; 2-6 to 2-8: Connecting tubes; 3-1: X-axis; 3-2: Y-axis; 3-3: Liquid injection module Z-axis; 3-4: Pipetting apparatus Z-axis; 3-5: Pipette body; 3-6: Pipette tip; 4-1: Reactor/reaction tube; 4-2: Reaction tube adapter sleeve; 4-3: Heat transfer fan; 4-4: Motion guide rail 1; 4-5: Motion guide rail 2; 4-6: Heating rod; 4-7: Reaction tube seat; 4-8: Shaking motor; 4-9: First sealing ring; 4-10: Second sealing ring; 4-11: Waste liquid discharge port; 4-12: Positive pressure gas supply port; 5-1-1 to 5-1-10: openings No. 1 to No. 10 of liquid injection port No. 1 (corresponding to reaction tube No. 1); 5-2-1: opening No. 1 of liquid injection port No. 2 (corresponding to reaction tube No. 2); 5-2-10: opening No. 10 of liquid injection port No. 2 (corresponding to reaction tube No. 2); 5-6-1: opening No. 1 of liquid injection port No. 6 (corresponding to reaction tube No. 6); 5-6-10: opening No. 10 of liquid injection port No. 6 (corresponding to reaction tube No. 6).

### Detailed Description of the Embodiments

A further detailed description is provided below of the specific embodiments of the present application. The following detailed description is provided to illustrate the content of the present application, but should not be construed as limiting the scope of the present application.

The "ranges" disclosed herein are defined in the form of lower limits and upper limits; a given range is defined by selecting a lower limit and/or an upper limit, which delimit the boundaries of the specific range. The range defined in this way can include or exclude end values, and can be combined arbitrarily; that is, any lower limit can be combined with any upper limit to form a range. For example, if ranges of 60-120 and 80-110 are listed for a specific parameter, it is understood that the ranges of 60-110 and 80-120 are also contemplated. Furthermore, if the minimum range values 1 and 2 are listed, and if the maximum range values 3, 4 and 5 are listed, then the following ranges can all be expected: 1-3, 1-4, 1-5, 2-3, 2-4 and 2-5. Herein, unless otherwise specified, a numerical range expressed as "a-b" is an abbreviated representation denoting any combination of real numbers falling between a and b, where both a and b are real numbers. For example, the numerical range "0-5" means that all real numbers between "0-5" have been listed herein; "0-5" is just a shortened representation of these numerical combinations. In addition, when a parameter is expressed as an integer ≥2, this shall be equivalent to disclosing that the parameter may be, for example, integers such as 2, 3, 4, 5, 6, 7, 8, 9, 10, 11, 12, and so forth.

Unless otherwise specified, all embodiments and preferred embodiments mentioned herein can be combined to form new technical solutions.

Unless otherwise specified, all technical features and preferred features mentioned herein can be combined to form new technical solutions.

Unless otherwise specified, the terms "comprising" and "including" mentioned herein shall denote either an open-ended or a closed-ended meaning. For example, the "comprising" and "including" may mean that they can further include other components not listed, or they can only include the components listed.

In the description herein, it should be noted that unless otherwise specified, "and more" and "and less" include the number itself; in the phrase "one or more", the meaning of "more" refers to two or more.

In the description herein, unless otherwise stated, the term "or" is inclusive. For example, the phrase "A or B" means "A, B, or both A and B". More specifically, any of the following conditions satisfy condition "A or B": A is true (or exists) and B is false (or does not exist); A is false (or does not exist) and B is true (or exists); or both A and B are true (or exist).

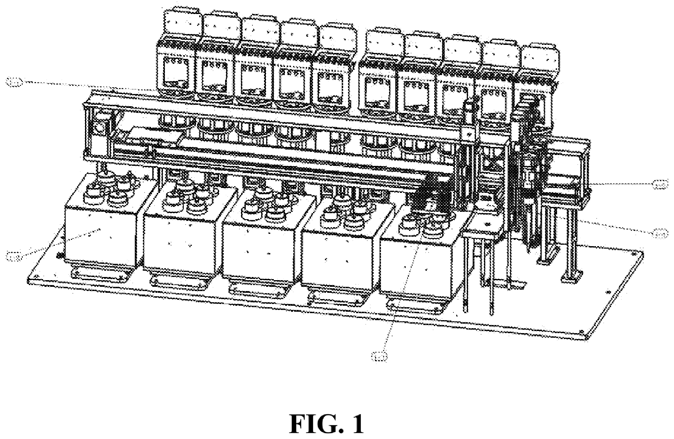

The fully automatic oligonucleotide synthesis device disclosed herein mainly comprises: a pipeline liquid feeding system, a reaction system and a control system. The pipeline liquid feeding system is used to supply various reagents required for oligonucleotide reactions from their respective storage devices, such as reagent reservoirs (e.g., reagent bottles), to the reaction system via pipelines. These reagents include, but are not limited to, washing agents, deprotecting agents, neutralizing agents, coupling units, etc. As shown in Fig. 1, the pipeline liquid feeding system comprises a pumping module 1-1, a liquid injection module 1-3, and a moving module 1-4. The reagent reservoir (not shown in Fig. 1) is connected to the pumping module 1-1 via a pipeline, and the pumping module 1-1 is connected to the liquid injection module 1-3 via a pipeline. To avoid affecting observation, the pipelines are not shown in Fig. 1. In some embodiments, in addition to the pipeline liquid feeding system, the fully automatic oligonucleotide synthesis device of the present disclosure further comprises a non-pipeline liquid feeding system, the non-pipeline liquid feeding system comprises a pipetting apparatus, and the pipetting apparatus directly removes reagents from a reagent storage device and injects them into the reaction system, such as into a reactor within the reaction system. In other words, there is no pipeline connection between the pipetting apparatus and the reaction system, which is especially advantageous for reagents used in small quantities (such as coupling units) and avoids reagent waste. For example, one specific form of pipetting apparatus can be a pipette, such as pipette 1-5 as shown in Fig. 1. Fig. 1 also shows a reaction system, which exemplarily comprises 5 reaction modules 1-2. The pumping module 1-1, reaction module 1-2, liquid injection module 1-3, moving module 1-4, and pipette 1-5 will be described in more detail below with reference to the corresponding accompanying drawings.

The control system (not shown) is in communication connection with the pipeline liquid feeding system, the non-pipeline liquid feeding system, and the reaction system. The communication connection includes a wired connection and/or a wireless connection. Under the control of the control system, the pumping module 1-1 automatically draws reagents from the reagent reservoir and then delivers them to the liquid injection module 1-3. The liquid injection module 1-3 is connected to the moving module 1-4, for example, it is mounted on the moving module 1-4. Under the control of the control system, the moving module 1-4 automatically moves the liquid injection module 1-3 to a position corresponding to the reaction module 1-2 requiring reagent injection, for example, above the reaction module 1-2, thereby injecting the reagents into the reactor in the reaction module 1-2. The pipetting apparatus, such as pipette 1-5, is also connected to the moving module 1-4. Under the control of the control system, the moving module 1-4 automatically moves the pipetting apparatus to the reagent reservoir, draws the reagent from the reagent reservoir, and then moves the pipetting apparatus to a position corresponding to the reaction module 1-2 requiring reagent injection, for example, above the reaction module 1-2, thereby injecting the drawn reagent into the reactor in the reaction module 1-2.

Although 10 pumping modules 1-1 are shown in Fig. 1, this is merely for illustrative purposes. The number of pumping modules 1-1 contained in the fully automatic oligonucleotide synthesis device of the present disclosure is not particularly limited, for example, there may be less than 10 pumping modules 1-1 or more than 10 pumping modules 1-1. In some embodiments, the number of pumping modules 1-1 may be configured according to the number of reagent types required for the synthesis of oligonucleotides, with each pumping module 1-1 delivering only one type of reagent. For example, if 8 types of reagents are required for oligonucleotide synthesis, 8 pumping modules 1-1 may be configured. If 2 out of these 8 types of reagents need to be transferred by the pipetting apparatus, 6 pumping modules 1-1 may be configured. Although the fully automated oligonucleotide synthesis device disclosed herein may have the number of pumping modules and pipetting apparatuses customized according to the specific oligonucleotides to be synthesized, a universal number of pumping modules and pipetting apparatuses may also be configured. For example, 12 pumping modules 1-1 may be configured. If a specific oligonucleotide synthesis reaction requires only 6 types reagents to be pumped, then only 6 pumping modules 1-1 out of the 12 pumping modules 1-1 need to be activated. Similarly, a plurality of (e.g., 6) pipetting apparatuses may be configured. If a specific oligonucleotide synthesis reaction requires only 2 types of reagents to be transferred using the pipetting apparatus, then only 2 pipetting apparatuses out of the 6 pipetting apparatuses need to be used. Therefore, any number of pumping modules and any number of pipetting apparatuses are included within the scope of protection claimed in the present application. In a preferred embodiment, each pumping module and each pipetting apparatus delivers only one type of reagent, thereby avoiding cross-contamination caused by the same pumping module or pipetting apparatus delivering different reagents.

Similarly, Fig. 1 shows 5 reaction modules, each of which comprises 6 reactors (reaction tubes). However, the number of reaction modules contained in the fully automatic oligonucleotide synthesis device disclosed herein, and the number of reactors contained in each reaction module, are not particularly limited. In some embodiments, the reaction system may comprise at least 1 reaction module; for example, 1-20 reaction modules, 2-15 reaction modules, 3-10 reaction modules, 4-9 reaction modules, 5-8 reaction modules, 6-7 reaction modules, such as 2, 3, 4, 5, 6 or more reaction modules. Similarly, each reaction module may comprise at least 1 reactor; for example, 1-20 reactors, 2-15 reactors, 3-10 reactors, 4-9 reactors, 5-8 reactors, 6-7 reactors, such as 2, 3, 4, 5, 6 or more reactors. Therefore, any number of reaction modules and any number of reactors are included within the scope of protection claimed in the present application. The more reaction modules and reactors there are, the larger the synthesis scale. Therefore, the fully automatic oligonucleotide synthesis device disclosed herein can be customized in terms of the number of reaction modules and reactors according to actual synthesis needs, or a general number of reaction modules and reactors may be configured to enable all or some of the reaction modules and reactors as needed. Since each pumping module and each pipetting apparatus delivers only one type of reagent, different reaction modules and/or reactors can perform the same synthesis reaction or different synthesis reactions. Therefore, the fully automatic oligonucleotide synthesis device disclosed herein can realize fully automatic oligonucleotide synthesis from laboratory scale to industrial scale, thus having great flexibility and significantly improving synthesis efficiency.

Similarly, Fig. 1 shows 1 injection module 1-3, but in other embodiments, a plurality of injection modules may be configured. Any number of liquid injection modules are included within the scope of protection claimed in the present application.

In the fully automatic oligonucleotide synthesis device disclosed herein, the pumping module 1-1, reaction module 1-2, liquid injection module 1-3, and pipetting apparatus (e.g., pipette 1-5) can be either fixed or detachable. In the detachable embodiments, only a portion of the pumping modules 1-1, reaction modules 1-2, liquid injection modules 1-3, and/or pipetting apparatuses (e.g., pipettes 1-5) may be mounted in the synthesis device as needed. The pumping module 1-1, reaction module 1-2, liquid injection module 1-3, moving module 1-4 and pipetting apparatus (e.g., pipette 1-5) are not limited to the arrangement shown in Fig. 1, but can be arranged in various ways as long as the objective of the present disclosure can be achieved.

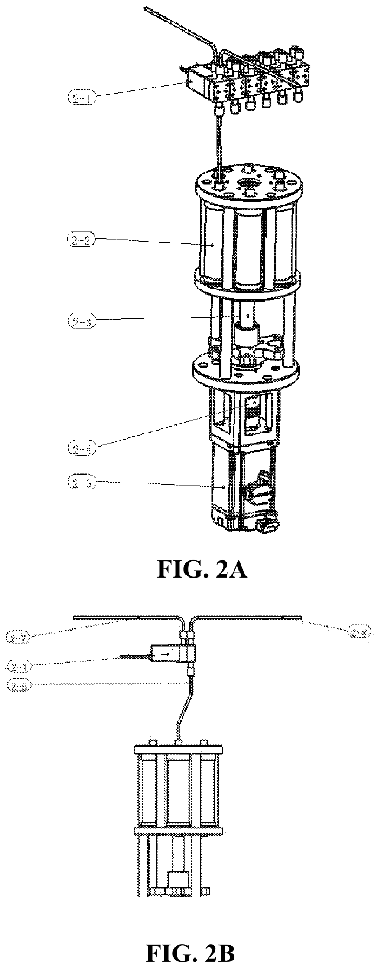

Fig. 2A is a schematic diagram of a pumping module according to some embodiments of the present disclosure. The pumping module comprises a solenoid valve 2-1 and an electric injector 2-2. The electric injector 2-2 is connected to the solenoid valve 2-1 via a pipeline. The solenoid valve 2-1 comprises an input port and an outout port (i.e., the two pipeline connection ports located at the top of the solenoid valve 2-1 as shown in the figure), where the input port is connected to the reagent reservoir via a pipeline and the output port is connected to the liquid injection module via a pipeline. The pumping module further comprises a drive component for driving the electric injector 2-2. For example, in the embodiment shown in Fig. 2, the pumping module further comprises a lead screw 2-3, a coupling 2-4, and a pumping motor 2-5. The pumping motor 2-5 drives the injector 2-2 through the coupling 2-4 and the lead screw 2-3. It first pulls the piston of the injector 2-2 outward, thereby drawing the reagent into the interior of the injector 2-2 through the input port of the solenoid valve 2-1. Then, the solenoid valve 2-1 switches channels, and the pumping motor 2-5 drives the injector 2-2 to push the piston of the injector 2-2 inward, thereby pumping the reagent out through the output port of the solenoid valve 2-1, thus realizing liquid suction and liquid feeding. The pumping module is in communication connection with the control system. For example, the solenoid valve 2-1 and the pumping motor 2-5 are connected to the control system to achieve automatic pumping.

Fig. 2A shows 6 solenoid valves 2-1 and 6 electric injectors 2-2. To avoid affecting the observation, only one solenoid valve is shown connected to an electric injector via a pipeline, and only the two pipelines above the solenoid valve are shown, one leading to the reagent reservoir and the other to the liquid injection module. However, it should be understood that in some embodiments of the fully automatic oligonucleotide synthesis device disclosed herein, more or fewer solenoid valves and electric injectors may be included. For example, it may include 1-10, 2-9, 3-8, 4-7, or 5-6 solenoid valves and electric injectors. The fully automatic oligonucleotide synthesis device disclosed herein can be customized in terms of the number of solenoid valves and electric injectors according to actual synthesis needs, or a general number of solenoid valves and electric injectors may be configured to activate all or some of the solenoid valves and electric injectors as needed (when a solenoid valve is closed, its corresponding electric injector will also stop its liquid suction and liquid feeding functions). Any number of solenoid valves and any number of electric injectors are included within the scope of protection claimed in the present application. Typically, one solenoid valve 2-1 is connected to one electric injector 2-2 via a pipeline. In a pumping module, the pumping motor 2-5 drives all the electric injectors 2-2 via the lead screw 2-3 and the coupling 2-4. It should be understood that although Fig. 2A shows 6 solenoid valves connected in parallel, they may not be connected in parallel, but may be arranged in any other suitable manner. It should also be understood that although 6 electric injectors are shown arranged in a circular pattern in Fig. 2A, any other suitable arrangement may also be adopted.

Fig. 2B is a partial schematic diagram of a pumping module according to some embodiments of the present disclosure, which more specifically shows how the pipelines of the solenoid valve 2-1 are connected. In the illustrated embodiment, the connecting tube 2-6 is connected to the injector 2-2, the connecting tube 2-8 is connected to the reagent reservoir, and the connecting tube 2-7 is connected to the liquid injection module. In this case, the solenoid valve makes the connecting tube 2-8 in communication with the connecting tube 2-6 while the connecting tube 2-6 not in communication with the connecting tube 2-7. The pumping motor drives the injector 2-2 to pull the piston of the injector 2-2 outward, thereby drawing the reagent from the reagent reservoir through the connecting tube 2-8, the solenoid valve 2-1 and the connecting tube 2-6 into the interior of the injector 2-2. Then, the solenoid valve 2-1 switches channels to make the connecting tube 2-6 in communication with the connecting tube 2-7 while the connecting tube 2-6 not in communication with the connecting tube 2-8. Then, the pumping motor 2-5 drives the injector 2-2, causing the reagent drawn into the injector 2-2 to be pumped out through the solenoid valve 2-1 from the connecting tube 2-7. This allows for both liquid suction and liquid feeding.

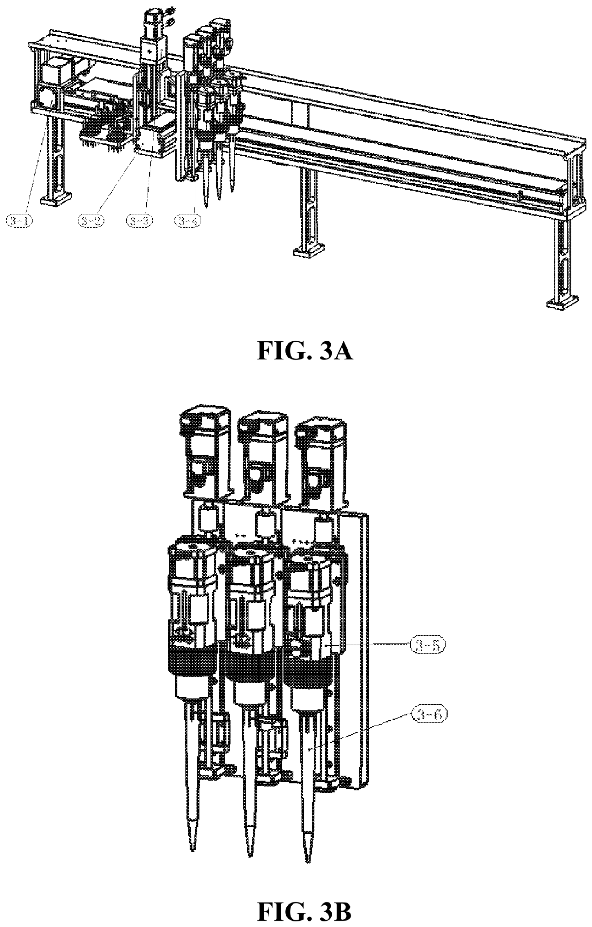

Fig. 3A is a schematic diagram of a moving module according to some embodiments of the present disclosure. In some embodiments, the moving module comprises a guide rail system for enabling the liquid injection module 1-3 and the pipetting apparatus, such as the pipette 1-5, to move three-dimensionally, thereby moving to the injection position under the control of a preset program. The guide rail system may comprise a first guide rail, a second guide rail, and a third guide rail that are perpendicular to each other. For ease of description, the guide rail 3-1 along the X-axis in the figure can be called the first guide rail, and the guide rail 3-2 along the Y-axis can be called the second guide rail. The third guide rail along the Z-axis direction may comprise two sets of guide rails: the third guide rail of the liquid injection module along the Z-axis direction 3-3 of the liquid injection module and the third guide rail of the pipetting apparatus along the Z-axis direction 3-4 of the pipetting apparatus. In some embodiments, the second guide rail may be configured to move horizontally along the first guide rail (i.e., along the X-axis direction); the third guide rail (including the third guide rail of the liquid injection module and the third guide rail of the pipetting apparatus) may be mounted on the second guide rail and configured to move horizontally along the second guide rail (i.e., along the Y-axis direction). The liquid injection module is mounted on the third guide rail of the liquid injection module and configured to move vertically up and down along the third guide rail of the liquid injection module (i.e., along the Z-axis direction). Similarly, the pipetting apparatus (e.g., the pipette 1-5 shown in the figure) is mounted on the third guide rail of the pipetting apparatus and configured to move vertically up and down along the third guide rail of the pipetting apparatus (i.e., along the Z-axis direction). The terms "first," "second," "third," "X-axis," "Y-axis," and "Z-axis" used above are merely for descriptive convenience and do not limit any specific orientation. For example, the guide rail along the Y-axis can be called the "first guide rail", while the guide rails along the other two directions can be called the "second guide rail" and the "third guide rail", respectively. The first guide rail, the second guide rail, and the third guide rail may each comprise a set of guide rails, for example, each may comprise one or more guide rails. The form of these guide rails is not particularly limited; they can be guide rails known in the art, as long as they can enable three-dimensional movement of the liquid injection module and the pipetting apparatus. Fig. 3A shows only one set of first guide rails, one set of second guide rails, one set of third guide rails for the liquid injection module, and one set of third guide rails for the pipetting apparatus. However, embodiments with multiple sets of first guide rails, multiple sets of second guide rails, multiple sets of third guide rails for the liquid injection module, and/or multiple sets of third guide rails for the pipetting apparatus are also included within the scope of protection claimed in the present application. In the specification and claims of the application, where the term "third guide rail" is not limited by the expressions "liquid injection module" or "pipetting apparatus", it refers to the third guide rail of the liquid injection module and/or the third guide rail of the pipetting apparatus.

The moving module further comprises a drive apparatus (not shown), such as an electric drive apparatus, for driving the guide rail, the liquid injection module and the pipetting apparatus to move, thereby enabling three-dimensional movement of the liquid injection module and the pipetting apparatus. The mobile module is in communication connection with the control system, enabling automatic three-dimensional movement of the liquid injection module and pipetting apparatus under the control of the control system.

Fig. 3B is a schematic diagram of a pipette according to some embodiments of the present disclosure. It is an enlarged schematic diagram of the pipette 1-5 mounted on the moving module as shown in Fig. 3A. As shown in the figure, the pipette 1-5 comprises a pipette body 3-5 and a pipette tip 3-6. The pipette tip 3-6 is detachable. In use, it is tightly fitted onto the pipette with no gaps or air leakage at the contact interface. During pipetting, the pipette tip 3-6 is inserted below the liquid surface to create a vacuum inside the pipette, drawing the liquid reagent into the pipette tip 3-6 and retaining it inside the tip. Then, the pipette is moved to the liquid injection position via the moving module, dispensing the liquid reagent from the pipette tip 3-6 into the reactor, thus completing the pipetting process. The pipette can be a commercially available electronically controlled pipette, such as those commonly used in automatic liquid dispensers. The pipette tip can be placed in the tip box. When needed, the moving module automatically moves the pipette body to the tip box under the control of the control system and automatically retrieves the tip. The pipette can control the amount of liquid dispensed, and can dispense a fixed amount of liquid into one or more reactors at a time. Fig. 3A shows 3 pipettes, but the number of pipettes in the fully automatic oligonucleotide synthesis device of the present disclosure is not particularly limited. A specific number of pipettes can be customized as needed; alternatively, a standard number of pipettes may be configured, with all or some of the pipettes used as needed during the synthesis process. Liquid can be sequentially dispensed into a plurality of reactors using a single pipette, or simultaneously dispensed into a plurality of reactors using a plurality of pipettes. To avoid cross-contamination, it is preferable that one pipette is dedicated to dispensing only one type of reagent.

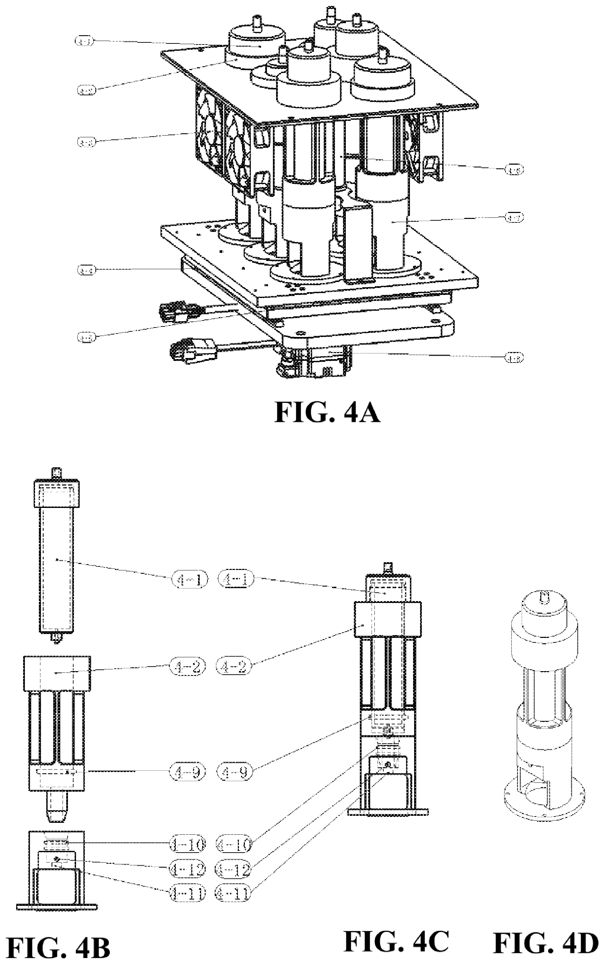

Fig. 4A is a schematic diagram of a reaction module according to some embodiments of the present disclosure. The reaction module comprises a reactor. In Fig. 4A, the reactor is in the form of reaction tube 4-1, which is cylindrical or substantially cylindrical. It should be understood that the reactor can also be of other shapes, such as cuboid, hexagonal, or other polygonal shapes, as long as they are suitable for the synthesis of oligonucleotides. In a preferred embodiment, the reactor is in the form of a reaction tube. As shown in Fig. 4A, the reaction tube 4-1 is mounted inside a reaction tube adapter sleeve 4-2. The reaction tube 4-1, which is mounted inside the reaction tube adapter sleeve 4-2, is placed on a reaction tube seat 4-7.

The reaction module may further comprise a shaking mechanism, and the shaking mechanism comprises a first set of motion guide rails 4-4 and a second set of motion guide rails 4-5. In some embodiments, the two sets of motion guide rails are perpendicular to each other. Each set of motion guide rails comprises at least one guide rail. In Fig. 4A, the first set of motion guide rails 4-4 comprises two guide rails, with only a portion of the guide rail on the right side of the figure shown. The second set of motion guide rails 4-5 also comprises two guide rails, of which only the guide rail on one side is shown and the guide rail on the other side is not shown. It should be understood that there is no particular limitation on the number of guide rails, and any number of guide rails are included within the scope of protection claimed in the present application. The first set of motion guide rails 4-4 is provided with a first support plate, and the second set of motion guide rails 4-5 is provided with a second support plate. The second set of motion guide rails 4-5 is located between the first support plate and the second support plate, and the reaction tube 4-1 is mounted on the second support plate. The shaking mechanism further comprises a shaking motor 4-8. Under the control of the control system, the shaking motor 4-8 drives the first support plate and the second support plate to move along the first set of motion guide rails 4-4 and the second set of motion guide rails 4-5, thereby causing the reaction tube 4-1 to shake regularly.

The reaction module may further comprise a heating and insulation apparatus, the heating and insulation apparatus comprises a heater (e.g., a heating rod 4-6 shown in Fig. 4A) and a heat transfer fan 4-3. The heating rod 4-6 is used to heat the reactors (reaction tubes 4-1 as shown in Fig. 4A) in the entire reaction module, and the heat transfer fan 4-3 makes the heat inside each reactor evenly distributed. The heating and insulation apparatus further comprises a temperature sensor for monitoring the temperature of the reactor. Under the control of the control system, the reaction module is capable of automatic heating and heat preservation, thereby achieving closed-loop temperature control.

The reaction module may further comprise inert gas pipes (not shown) connected in parallel with all reactors in the reaction module, and the inert gas supplied to each reactor can be controlled individually. Inert gases can include gases such as nitrogen, argon, and helium that can provide an inert atmosphere inside the reactor; nitrogen is commonly used. Dry nitrogen at a certain pressure is purged into the reaction tube; the filled nitrogen occupies the limited space inside the reaction tube and expels the limited air within the reaction tube to the outside, thereby achieving air isolation. The pressure of nitrogen shall not be excessively high, nor shall its flow rate be excessively fast; otherwise, the solids inside the reaction tube may be blown over and even adhered to the tube wall. Therefore, the pressure and flow rate of nitrogen are both set to be adjustable. Under the control of the control system, the pressure and flow rate of nitrogen can be automatically adjusted.

The reaction module may further comprise a negative pressure assembly (not shown). The negative pressure assembly is in communication with the liquid outlet at the bottom of the reactor, and the other end is connected to a vacuum chamber equipped with a vacuum pump. When the liquid is discharged from the reactor, the discharged liquid first goes to the vacuum chamber and then is discharged out of the device through the vacuum pump. The residual liquid and washing liquid after the reaction are pumped out of the reactor through the negative pressure assembly, thus achieving negative pressure drainage.

The present disclosure creates favorable reaction conditions through the shaking, heating and heat preservation, air isolation, and negative pressure drainage of the aforementioned components.

Fig. 4B is an exploded view of the reaction tube 4-1, the reaction tube adapter sleeve 4-2 and the reaction tube seat 4-7 according to some embodiments of the present disclosure; Fig. 4C is a schematic diagram of the assembly formed by assembling the reaction tube 4-1, the reaction tube adapter sleeve 4-2 and the reaction tube seat 4-7; and Fig. 4D is a perspective view of the assembly. By designing the reaction tube adapter sleeve 4-2, the reaction module of the present disclosure can be compatible with reaction tubes of different specifications. Specifically, the reaction module may be uniformly designed with a chamber of fixed specifications for accommodating reaction tubes; the external shape and dimensions of the reaction tube adapter sleeve may be designed to fit exactly into the chamber, while its internal shape and dimensions may be designed to have different specifications so as to accommodate reaction tubes of different specifications. For example, Fig. 1 and Fig. 4A each show three specifications of reaction tubes and their corresponding three specifications of reaction tube adapter sleeves. Reactor tubes 4-1 of different specifications can be placed on the device with the help of reaction tube adapter sleeves 4-2, and the device is compatible with all of them. Reaction tubes of different dimensions have their own matching adapter sleeves. The adapter sleeve is equipped with a first sealing ring 4-9. When the reaction tube is placed inside the adapter sleeve, the first sealing ring 4-9 plays a sealing role, enabling the reaction tube and the adapter sleeve to be sealed as an integrated whole. It should be understood that Fig. 4B only uses reaction tubes 4-1 and reaction tube adapter sleeves 4-2 as examples, and the corresponding reactor adapter sleeve may be designed for any reactor.

As shown in Figs. 4B and 4C, the reaction tube seat 4-7 is equipped with a positive pressure gas supply port 4-12 and a waste liquid discharge port 4-11, and the opening and closing of the two ports are controlled by two solenoid valves respectively. During the reaction, the solenoid valve at the waste liquid discharge port 4-11 is closed, while the gas supply port 4-12 is opened, allowing positive pressure gas to enter the pipe seat. A second sealing ring 4-10 is mounted inside the pipe seat. By virtue of the action of the second sealing ring 4-10, the gas flows upward into the reaction tube adapter sleeve; further, by virtue of the action of the first sealing ring 4-9, the gas smoothly enters the interior of the reaction tube. After the reaction is completed, the gas supply port 4-12 is closed, and the waste liquid discharge port 4-11 connected to the vacuum is opened, allowing the waste liquid to flow out through the waste liquid discharge port 4-11; the port is then closed again after all the waste liquid has been completely discharged.

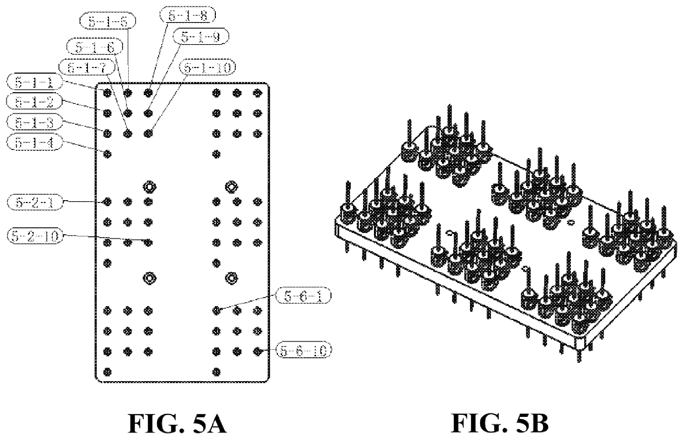

Fig. 5A is a schematic diagram showing the distribution of liquid injection ports according to some embodiments of the present disclosure. For illustrative purposes only, this figure shows 6 liquid injection ports, each containing 10 openings. The 6 liquid injection ports correspond to 6 reactors (reaction tubes) in one reaction module. Each liquid injection port corresponds to the inlet of one reactor (reaction tube), so that reagents passing through each opening in the liquid injection port can enter the reactor (reaction tube) through the inlet. The 10 openings allow for the injection of 10 different types of reagents. Using the liquid injection module shown in the figure, the same reagent can be injected into up to 6 reactors simultaneously. For example, as shown in Fig. 5A, if the 10 openings in the first liquid injection port corresponding to the first reaction tube (reaction tube No. 1) are numbered sequentially as openings No. 5-1-1 to No. 5-1-10, they can be used for injecting the first reagent (reagent No. 1) to the tenth reagent (reagent No. 10), respectively. Similarly, the second injection port marked in the figure has openings No. 5-2-1 and No. 5-2-10 corresponding to the second reaction tube (reaction tube No. 2), and the sixth injection port has openings No. 5-6-1 and No. 5-6-10 corresponding to the sixth reaction tube (reaction tube No. 6) (other openings can be numbered similarly, but to avoid interfering with observation, the other opening numbers are not marked in the figure). Openings No. 5-2-1 and No. 5-6-1 can be used for injecting reagent No. 1, while openings No. 5-2-10 and No. 5-6-10 can be used for injecting reagent No. 10.

Fig. 5B is a schematic diagram of a liquid injection module according to some embodiments of the present disclosure. As shown in the figure, a liquid injection connector is inserted into each opening, the upper end of the connector is connected to a reagent delivery pipeline, and the lower end is a cylindrical or needle-shaped liquid injection tube. It should be understood that a liquid injection module may comprise more than 6 or fewer than 6 liquid injection ports, each liquid injection port may comprise more than 10 or fewer than 10 openings, and correspondingly may comprise more than 10 or fewer than 10 liquid injection connectors. The openings in each liquid injection port are not necessarily arranged as shown in Fig. 5A. The arrangement of the openings is not critical and is not particularly restricted, as long as the liquid injection tube of each liquid injection connector can be inserted into the reactor inlet when the liquid injection module is in the liquid injection position. Any number of liquid injection ports, any number of openings, and any arrangement mode of the openings shall fall within the scope of protection claimed in the present application. It should be understood that for a specific oligonucleotide synthesis reaction, it is not necessary to put every liquid injection port and/or every opening into use. Therefore, in terms of the number of liquid injection ports and openings, it can be customized for specific oligonucleotide synthesis reactions, or a universal liquid injection module may be configured.

In a preferred embodiment of the fully automatic oligonucleotide synthesis device disclosed herein, each type of reagent is delivered to the reactor via a separate route. For non-pipeline liquid feeding systems, such as those using pipettes to transfer reagents, each type of reagent requires a separate pipette. For pipeline liquid feeding systems, for example, for a certain oligonucleotide synthesis reaction, if each reaction module uses 6 reactors as shown in Figs. 1 and 4A, and each reactor performs the same synthesis reaction, then each reagent needs to be divided into 6 streams to enter these 6 reactors. In one embodiment, the 6 pipelines can be connected to the same reagent reservoir, the other end of each pipeline can be connected to a solenoid valve in the pumping module as shown in Fig. 2A (as shown in Fig. 2B), the outlet pipeline of each solenoid valve is connected to a liquid injection connector in a liquid injection port as shown in Fig. 5B, such that the outlet pipelines of the 6 solenoid valves are respectively connected to one liquid injection connector in the 6 liquid injection ports, and in this way, the same reagent can be simultaneously and separately injected into the 6 reactors. In this embodiment, a reagent reservoir may comprise 6 outlets, each connected to a single pipeline; alternatively, the 6 pipelines may be grouped together and connected to a single outlet of the reagent reservoir. Of course, other connection modes can also be used. All these connection modes shall fall within the scope of protection claimed in the present application.

For the synthesis reactions enumerated above, in another embodiment, 1 pipeline can be connected to a reagent reservoir, and the other end of the pipeline can be connected to a solenoid valve in the pumping module as shown in Fig. 2A (as illustrated in Fig. 2B). The outlet pipeline of the solenoid valve is connected to a flow divider valve (e.g., a flow-dividing solenoid valve, not shown), which splits the reagent into 6 paths. Each path of the pipeline is connected to a liquid injection connector in a liquid injection port as shown in Fig. 5B, thereby enabling the same reagent to be simultaneously and separately injected into the 6 reactors.

The fully automatic oligonucleotide synthesis device disclosed herein further comprises a control system. The control system comprises commercially available hardware (such as memory, monitor, processor) and software (such as programming software). Those skilled in the art may perform programming according to the control functions to be implemented by the synthesis device disclosed herein.

The method for synthesizing oligonucleotides by using a fully automatic oligonucleotide synthesis device disclosed herein comprises:
a. washing a reactor;
b. adding a deprotecting agent to the reactor to carry out a deprotection reaction;
c. washing the reactor;
d. adding a neutralizing agent to the reactor to carry out a neutralization reaction;
e. washing the reactor;
f. adding a coupling unit to the reactor;
g. carrying out a coupling reaction,
wherein under the control of a control system, steps a to h are executed cyclically until the desired oligonucleotide is obtained, the method comprising, under the control of the control system, automatically moving a liquid injection module using a moving module to inject the reagent into the reactor. For reagents with small quantities or those that are unstable, the method for synthesizing oligonucleotides further comprises, under the control of the control system, using the pipetting apparatus to transfer and inject the reagent into the reactor.

In the method for synthesizing oligonucleotides by using the fully automatic oligonucleotide synthesis device disclosed herein, the fully automatic oligonucleotide synthesis device comprises a plurality of reaction modules, after step f is completed in one reaction module and during the execution of step g, steps a to f are performed on the next reaction module until steps a to f are completed in all the required number of reaction modules. This can significantly improve synthesis efficiency.

One or more of the processes of washing, deprotection reaction, neutralization reaction, and coupling reaction involve one or more of the following operations under the control of the control system:
shaking the reactor;
heating the reactor to a predetermined temperature and maintaining it at that predetermined temperature;
blowing nitrogen into the reactor for isolation from air.

In the present disclosure, oligonucleotides include, but are not limited to, phosphorodiamidate morpholino oligomers.

In step a, washing may be performed first with dichloromethane (DCM), followed by washing with 30% trifluoroethanol (TFE)/dichloromethane (DCM). These washing agents can be added using the liquid injection module. In some embodiments, they are used to wash for 30 s to 5 min, for example, 30 s to 4 min, 1 min to 3 min.

In step b, the deprotecting agent may be a solution of trifluoroacetic acid (TFA) and 4-cyanopyridine in trifluoroethanol (TFE)/dichloromethane (CYTFA). The deprotecting agent can be added using the liquid injection module. The deprotection reaction lasts 10 to 20 min, for example, 12 to 18 min, 14 to 16 min, or 15 min.

In step c, the washing can be performed using dichloromethane (DCM). The washing agent can be added using the liquid injection module. The washing can be performed for 30 s to 5 min, for example, 30 s to 4 min, 1 min to 3 min.

In step d, the neutralizing agent is a mixed solution of N,N-diisopropylethylamine (DIEA), isopropanol and dichloromethane. The neutralizing agent can be added using the liquid injection module. The neutralization reaction can be performed for 30 s to 5 min, for example, 30 s to 4 min, 1 min to 3 min.

In step e, washing may be performed first with dichloromethane, followed by washing with 1,3-dimethyl-2-imidazolidinone (DMI). The washing agent can be added using the liquid injection module. Each washing can be performed for 30 s to 5 min, for example, 30 s to 4 min, 1 min to 3 min.

In step f, a 10% by volume solution of N-ethylmorpholine (NEM) in 1,3-dimethyl-2-imidazolidinone (DMI) and a 0.1M solution of phosphorodiamidate morpholino oligomer (PMO) monomer in 1,3-dimethyl-2-imidazolidinone (DMI) may be added to the reactor. An exemplary PMO monomer is shown below:

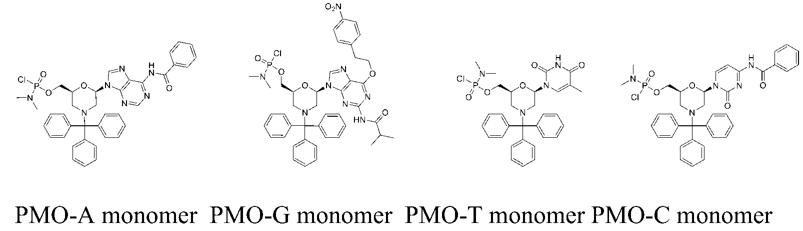

In some embodiments, the PMO can be represented by the following general formula: 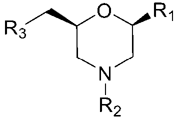
where R₁ represents the modified form of natural purine and pyrimidine bases (e.g., adenine, uracil, guanine, cytosine, and thymine), modified analogs thereof, and protected derivatives thereof;
R₂ represents hydrogen or triphenylmethyl, etc.;
R₃ represents an amide or imine, which can be represented by a group as shown in the following general formula: 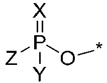
where X represents oxygen or sulfur; Y represents dialkylamino or alkoxy; Z represents chlorine.

They can all be added using the pipetting apparatus.

In step g, the reaction may last for 2 to 4 h, for example, 3 h.

The oligonucleotide synthesis device disclosed herein has the following beneficial effects:
(1) The oligonucleotide synthesis device disclosed herein improves the quality of chemical synthesis. The present disclosure proposes a technique for independent multi-channel liquid feeding to a single reaction tube, preventing cross-contamination of residual reagents caused by shared pipelines, thereby comprehensively improving the synthesis quality in all reaction tubes. The present disclosure uses a pipetting apparatus (pipette) to transfer monomers, replacing the traditional steel needle liquid feeding method, which fundamentally addresses the issues affecting synthesis quality such as liquid hanging on steel needles and crystallization on steel needles.
(2) The oligonucleotide synthesis device disclosed herein improves the reaction efficiency of chemical synthesis. The present disclosure achieves full, thorough and sufficient stirring inside the reaction tubes by virtue of the functions of inert gas stirring, parallel shaking and constant-temperature gas bath throughout the entire reaction process, thereby fundamentally addressing the problem of low reaction efficiency in the prior art caused by factors such as uneven mixing of reaction reagents and reagent deterioration due to air ingress.
(3) The oligonucleotide synthesis device disclosed herein achieves consistency in reaction quality within a plurality of reaction tubes. By programming and designing the entire production process, a high degree of automation can be achieved, ensuring that the entire process follows a predetermined procedure and reducing the probability of human errors during production. The present disclosure utilizes high-precision liquid feeding and drainage modules as well as adjustable independent gas inlet pipelines to ensure that each reaction tube is supplied with equal amounts of reaction reagents and inert gas, and that the reaction reagents can uniformly and synchronously enter each reaction tube. Coupled with the concurrent constant-temperature air bath and shaking functions, it guarantees consistent contact time and stirring speed of the reaction reagents in each reaction tube, while also ensuring uniform synthesis quality across all reaction tubes. Uniform synthesis quality within the reaction tube ensures the integrity and accuracy of all fragments during downstream synthesis, thereby guaranteeing the quality and efficiency of the synthesis. The term "fully automatic" as used herein refers to the automatic execution of various operations under the control of the control system, including liquid acquisition from reagent reservoirs, reagent delivery in pipelines, reagent injection into reactors, heating of reactors (including the operation of heating rods and fans) and shaking, delivery of inert gas, and discharge of waste liquid. However, this does not exclude necessary manual interventions such as manual reagent preparation and manual parameter input.
(4) The oligonucleotide synthesis device disclosed herein achieves high-quality and high-efficiency oligonucleotide synthesis in terms of liquid feeding method, chamber size, liquid discharge method and stirring method, and has groundbreaking innovative value for high-throughput column-based synthesizers.

### Examples

The present disclosure will be described in more detail below through specific examples. The following examples are for illustrative purposes only and are not intended to limit the present disclosure in any way. Those of skill in the art will readily recognize a variety of noncritical parameters which can be changed or modified to yield essentially the same results.

Unless otherwise specified, all reagents and materials used in the following examples are commercially available or synthesized.

### Example 1 Fully automatic synthesis device for phosphorodiamidate morpholino oligomers

The fully automated synthesis device for phosphorodiamidate morpholino oligomers in this example comprises 10 pumping modules, thus it can pump up to 10 reagents. Each pumping module comprises 6 solenoid valves and 6 electric injectors. The reaction system comprises 5 reaction modules, each containing 6 reaction tubes, for a total of 30 reaction tubes available for simultaneous use. The device comprises 3 pipettes and a pipette tip box containing the required pipette tips. Each pipette tip corresponds to a reagent bottle, and the 3 pipettes can dispense liquid simultaneously. The device comprises a liquid injection module with 6 liquid injection ports, each including 10 openings and 10 corresponding liquid injection connectors (as shown in Figs. 5A and 5B). The number of liquid injection ports corresponds to (i.e., is equal to) the number of reaction tubes; the number of openings (i.e., the number of liquid injection connectors) corresponds to (i.e., is equal to) the number of pumping modules, and also corresponds to (i.e., is equal to) the maximum number of reagent types that can be pumped. This ensures that each reagent is delivered to one reaction tube via an independent pipeline.

### Example 2 Fully automatic synthesis of phosphorodiamidate morpholino oligomers

Phosphorodiamidate morpholino oligomers were synthesized using the fully automated synthesis device for phosphorodiamidate morpholino oligomers in Example 1. The specific steps are as follows:
(1) Set the operating procedure on the control system.
(2) Transfer the swollen resin into the reaction tube through the feed inlet for use as a synthesis carrier of phosphorodiamidate morpholino oligomers; prepare various reagents.
(3) Input the required parameters, including time, temperature, shaking frequency and amplitude, and the amount of each reagent added.
(4) Input the oligonucleotide sequence information to be synthesized, the main cycle length, and the sub-cycle length, save it as a synthesis scheme, and click the start button to start the automatic operation. At the same time, control the system to execute the set parameters and track the device status and handle abnormalities.

The program runs as follows:
a. Washing with DCM:
   The moving module drove the liquid injection module to move, simultaneously adding DCM into the 6 reaction tubes of a reaction module. After the addition was completed, the moving module was lifted, the reaction module was activated to perform shaking according to the preset parameters. Meanwhile, the heating apparatus was turned on for heating to the set temperature (25°C), and nitrogen was purged for isolation from air. Shaking was continuously performed for 1 min according to the preset time, nitrogen purging was stopped, and negative pressure was generated by a vacuum pump to drain the liquid in the reaction tubes into the waste liquid barrel.
b. Washing with 30%TFE/DCM:
   The moving module drove the liquid injection module to move, simultaneously adding 30%TFE/DCM into the 6 reaction tubes of the reaction module. After the addition was completed, the moving module was lifted, the reaction module was activated to perform shaking according to the preset parameters. Meanwhile, the heating apparatus was turned on for heating to the set temperature (25°C), and nitrogen was purged for isolation from air. Shaking was continuously performed for 1 min according to the preset time, nitrogen purging was stopped, and negative pressure was generated by a vacuum pump to drain the liquid in the reaction tubes into the waste liquid barrel. This step was repeated twice.
c. Deprotection:
   The moving module drove the liquid injection module to move, simultaneously adding CYTFA solution into the 6 reaction tubes of the reaction module. After the addition was completed, the moving module was lifted, the reaction module was activated to perform shaking according to the preset parameters. The heating apparatus was turned on for heating to the set temperature (25°C), and nitrogen was purged for isolation from air. Shaking was continuously performed for 15 min according to the preset time, nitrogen purging was stopped, and negative pressure was generated by a vacuum pump to drain the liquid in the reaction tubes into the waste liquid barrel. This step was repeated twice.
d. Washing with DCM:
   The moving module drove the liquid injection module to move, simultaneously adding DCM into the 6 reaction tubes of the reaction module. After the addition was completed, the moving module was lifted, the reaction module was activated to perform shaking according to the preset parameters. The heating apparatus was turned on for heating to the set temperature (25°C), and nitrogen was purged for isolation from air. Shaking was continuously performed for 1 min according to the preset time, nitrogen purging was stopped, and negative pressure was generated by a vacuum pump to drain the liquid in the reaction tubes into the waste liquid barrel.
e. Neutralization:
   The moving module drove the liquid injection module to move, simultaneously adding the neutralizing solution into the 6 reaction tubes of the reaction module. (a mixed solution of N,N-diisopropylethylamine, isopropanol and dichloromethane). After the addition was completed, the moving module was lifted, the reaction module was activated to perform shaking according to the preset parameters. The heating apparatus was turned on for heating to the set temperature (25°C), and nitrogen was purged for isolation from air. Shaking was continuously performed for 1 min according to the preset time, nitrogen purging was stopped, and negative pressure was generated by a vacuum pump to drain the liquid in the reaction tubes into the waste liquid barrel. This step was repeated twice.
f. Washing with DCM:
   The moving module drove the liquid injection module to move, simultaneously adding DCM into the 6 reaction tubes of the reaction module. After the addition was completed, the moving module was lifted, the reaction module was activated to perform shaking according to the preset parameters. The heating apparatus was turned on for heating to the set temperature (25°C), and nitrogen was purged for isolation from air. Shaking was continuously performed for 1 min according to the preset time, nitrogen purging was stopped, and negative pressure was generated by a vacuum pump to drain the liquid in the reaction tubes into the waste liquid barrel. This step was repeated twice.
g. Washing with DMI:
   The moving module drove the liquid injection module to move, simultaneously adding DMI into the 6 reaction tubes of the reaction module. After the addition was completed, the moving module was lifted, the reaction module was activated to perform shaking according to the preset parameters. The heating apparatus was turned on for heating to the set temperature (25°C), and nitrogen was purged for isolation from air. Shaking was continuously performed for 1 min according to the preset time, nitrogen purging was stopped, and negative pressure was generated by a vacuum pump to drain the liquid in the reaction tubes into the waste liquid barrel.
h. Reaction:
   The moving module drives the pipette to transfer 10% NEM/DMI (V:V) solution into the reaction tube. According to the synthesis scheme, different PMO monomer solutions were transferred using a pipette and added to the corresponding reaction tubes.

After the addition was completed, the moving module was lifted, the reaction module was activated to perform shaking according to the preset parameters. The heating apparatus was turned on for heating to the set temperature (35°C), and nitrogen was purged for isolation from air. Shaking was continuously performed for 3 h according to the preset time. After the reaction was completed, nitrogen purging was stopped, and negative pressure was generated by a vacuum pump to drain the liquid in the reaction tubes into the waste liquid barrel.

The above steps constituted a sub-cycle. After completing the set number of sub-cycles, a prompt would appear indicating that reagents need to be prepared. After the required reagents had been manually prepared, the continue operation button was clicked, and the reaction device performed the preset sub-cycles in accordance with the input sequence information. The sub-cycles were repeated until the main cycle completed the synthesis, the display panel prompted the completion of synthesis, and the sample was taken out.

The sample was removed and washed with 1,3-dimethylpyrrolidone (NMP). The sample was then placed in a reactor and a solution of 1,8-diazabicyclo[5.4.0]undec-7-ene (DBU) and dithiothreitol (DTT) in 1,3-dimethylpyrrolidone (NMP) was added. The mixture was reacted at 35°C for 5 h to cleave the synthetic phosphorodiamidate morpholino oligomer from the carrier. The mixture was then filtered, and the filtrate was collected. The filtrate was added to a sealed container containing 25% ammonia and allowed to react at 55°C for 16 h to remove the protecting groups on the synthetic phosphorodiamidate morpholino oligomer.

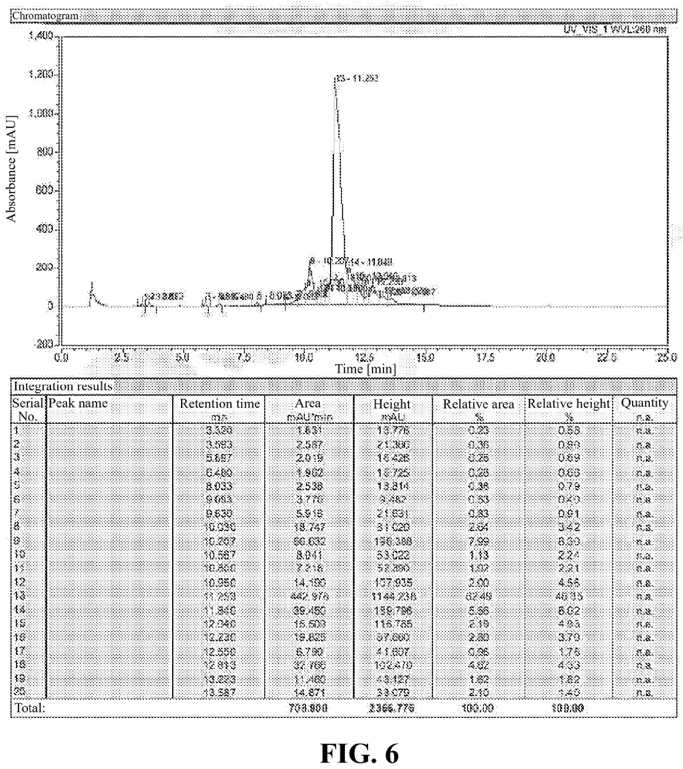

After the reaction was completed, the system was returned to room temperature, a sample was taken for chromatographic analysis, and the results are shown in Fig. 6, where the main peak with a retention time of 11.253 min was the phosphorodiamidate morpholino oligomer, and according to the integral results, the purity of the obtained crude phosphorodiamidate morpholino oligomer was 62-63%.

All documents mentioned in the present disclosure are incorporated by reference into the present application as if each individual document were specifically and individually incorporated by reference. Furthermore, it should be understood that after reading the foregoing teachings of the present disclosure, those skilled in the art can make various alterations or modifications to the present disclosure, and these equivalent forms also fall within the scope defined by the appended claims.

## Claims

1. A fully automatic oligonucleotide synthesis device, comprising a pipeline liquid feeding system, a reaction system and a control system, wherein the pipeline liquid feeding system comprises a pumping module (1-1), a moving module (1-4), and a liquid injection module (1-3), the liquid injection module (1-3) being mounted on the moving module (1-4), and the moving module (1-4) being configured to enable the liquid injection module (1-3) to move three-dimensionally to inject liquid into the reaction system; and the control system is in communication connection with the pipeline liquid feeding system and the reaction system and is used for controlling the liquid feeding and reaction; the device preferably further comprises a non-pipeline liquid feeding system, wherein the non-pipeline liquid feeding system comprises a pipetting apparatus (1-5), and the pipetting apparatus (1-5) is preferably mounted on the moving module (1-4) and is used for injecting the liquid into the reaction system.

2. The fully automatic oligonucleotide synthesis device according to claim 1, wherein the reaction system comprises at least one reaction module (1-2), preferably at least two reaction modules (1-2), such as 3, 4, 5 or 6 reaction modules (1-2); each reaction module (1-2) comprises at least one reactor, preferably at least two reactors (4-1), such as 3, 4, 5 or 6 reactors (4-1); each reactor (4-1) is optionally disposed in an adapter sleeve (4-2); the reaction module (1-2) preferably comprises a shaking mechanism, the shaking mechanism preferably comprises a motion guide rail, more preferably comprises two sets of motion guide rails (4-4, 4-5) configured to shake the reactor (4-1).

3. The fully automatic oligonucleotide synthesis device according to claim 2, wherein the device comprises one or more liquid injection modules (1-3), each liquid injection module (1-3) comprises a liquid injection port for injecting liquid into the reactor (4-1), preferably the number of liquid injection ports of each liquid injection module (1-3) is equal to the number of reactors (4-1) of each reaction module (1-2).

4. The fully automatic oligonucleotide synthesis device according to claim 1 or 2, wherein the moving module (1-4) comprises a first guide rail, a second guide rail and a third guide rail that are perpendicular to each other, the second guide rail is configured to move horizontally along the first guide rail; the third guide rail is mounted on the second guide rail and is configured to move horizontally along the second guide rail; the liquid injection module (1-3) is mounted on the third guide rail and is configured to move vertically up and down along the third guide rail.

5. The fully automatic oligonucleotide synthesis device according to claim 1 or 2, wherein the pumping module (1-1) comprises an electric injector (2-2) and a solenoid valve (2-1), the electric injector (2-2) and the solenoid valve (2-1) are connected via a pipeline, the solenoid valve (2-1) comprises an input port and an output port, the input port is connected to a reagent reservoir via a pipeline, and the output port is connected to the liquid injection module (1-3) via a pipeline;
the device preferably comprises a plurality of reagent reservoirs, wherein each of at least some of the reagent reservoirs is provided with one or more reagent outlets, and each reagent outlet is connected to the input port of a solenoid valve (2-1) via a pipeline;
preferably, at least one reagent reservoir comprises only one reagent outlet, the reagent outlet is connected to the input port of a solenoid valve (2-1), there is a flow divider between the output port of the solenoid valve (2-1) and the liquid injection module (1-3), the flow divider comprises a flow divider inlet and a plurality of flow divider outlets, the flow divider inlet is connected to the output port of the solenoid valve (2-1) via a pipeline, and each flow divider outlet is connected to a liquid injection port via a pipeline;
preferably, at least one of the plurality of reagent reservoirs is configured to allow the pipetting apparatus (1-5) to transfer reagents.

6. A method for synthesizing oligonucleotides by using a fully automatic oligonucleotide synthesis device, comprising:
a. washing a reactor (4-1);
b. adding a deprotecting agent to the reactor (4-1) to carry out a deprotection reaction;
c. washing the reactor (4-1);
d. adding a neutralizing agent to the reactor (4-1) to carry out a neutralization reaction;
e. washing the reactor (4-1);
f. adding a coupling unit to the reactor (4-1);
g. carrying out a coupling reaction,
wherein under the control of a control system, steps a to h are executed cyclically until the desired oligonucleotide is obtained, the method comprising, under the control of the control system, moving a liquid injection module (1-3) using a moving module (1-4) to inject the reagent into the reactor (4-1), preferably further comprising, under the control of the control system, using the pipetting apparatus (1-5) to transfer the reagent into the reactor (4-1), the liquid injection module (1-3) and the pipetting apparatus (1-5) being used for injecting different reagents.

7. The method according to claim 6, wherein the fully automatic oligonucleotide synthesis device comprises a plurality of reaction modules (1-2), after step f is completed in one reaction module (1-2) and during the execution of step g, steps a to f are performed on the next reaction module (1-2) until steps a to f are completed in all the required number of reaction modules (1-2).

8. The method according to claim 7, wherein one or more of the processes of washing, deprotection reaction, neutralization reaction, and coupling reaction, preferably all of the processes, involve one or more of the following operations under the control of the control system:
shaking the reactor (4-1);
heating the reactor (4-1) to a predetermined temperature and maintaining it at that predetermined temperature;
blowing nitrogen into the reactor (4-1) for isolation from air.

9. The method according to claim 6, wherein the oligonucleotide is a phosphorodiamidate morpholino oligomer.

10. The method according to claim 9, comprising at least one of the following:
in step a, the reactor is washed with dichloromethane and 30% trifluoroethanol/ dichloromethane in sequence, preferably using the liquid injection module (1-3) for addition, and each washing is preferably carried out for 30 s to 5 min, for example, 1 min;
in step b, the deprotecting agent is a solution of trifluoroacetic acid and 4-cyanopyridine in trifluoroethanol/dichloromethane, preferably using the liquid injection module (1-3) for addition, and the reaction is preferably carried out for 10 to 20 min, for example, 15 min;
in step c, the reactor is washed with dichloromethane, preferably using the liquid injection module (1-3) for addition, and the washing is preferably carried out for 30 s to 5 min, for example, 1 min;
in step d, the neutralizing agent is a mixed solution of N,N-diisopropylethylamine, isopropanol and dichloromethane, preferably using the liquid injection module (1-3) for addition, and the reaction is preferably carried out for 30 s to 5 min, for example, 1 min;
in step e, the reactor is washed with dichloromethane and 1,3-dimethyl-2-imidazolidinone in sequence, preferably using the liquid injection module (1-3) for addition, and each washing is preferably carried out for 30 s to 5 min, for example, 1 min;
in step f, a 10% by volume solution of N-ethylmorpholine in 1,3-dimethyl-2-imidazolidinone and a 0.1M solution of a phosphorodiamidate morpholino oligomer monomer in 1,3-dimethyl-2-imidazolidinone are added to the reactor (4-1), preferably using the pipetting apparatus (1-5) for addition;
in step g, the reaction lasts for 2 to 4 h, for example, 3 h.
